# EUROPEAN PATENT SPECIFICATION

(11) **EP 2 653 181 B1**
(45) Date of publication and mention of the grant of the patent: **09.03.2016**
(21) Application number: 11849145.5
(22) Date of filing: 15.12.2011
(51) Int. Cl.: A61M 15/00, B01D 46/00

(54) **POWDER MEDICAMENT MOUTHPIECE AND APPLICATION**
PULVERARZNEIMITTELMUNDSTÜCK UND SEINE ANWENDUNG
EMBOUT BUCCAL DE MÉDICAMENT EN POUDRE ET SON UTILISATION

(30) Priority: 17.12.2010 CN 201010596111
(43) Date of publication of application: 23.10.2013
(73) Proprietor: Chen, Qingtang, Yueqing, Zhejiang 325606 (CN)
(72) Inventor: CHEN, Xin, Nanjing Jiangsu 210002 (CN); CHEN, Qingtang, Yueqing, Zhejiang 325606 (CN)
(74) Representative: ABG Patentes, S.L.
(86) International application number: PCT/CN2011/084070
(87) International publication number: WO 2012/079524

(56) References cited:
- EP-A1- 1 238 680
- WO-A1-87/06475
- WO-A1-02/089875
- WO-A1-2004/045687
- WO-A2-2009/022347
- CN-A- 1 215 999
- CN-A- 1 720 072
- CN-A- 101 032 640
- CN-U- 202 020 763
- CN-U- 202 028 009
- CN-U- 202 061 200
- CN-Y- 201 197 840
- US-A- 3 653 380
- US-A- 5 287 850
- US-A- 6 098 619

## Description

### Technical field

The present invention relates to a medical device, in particular to a medication powder nozzle and its application, used independently or together with other dry powder inhalers.

### Background of the invention

Inhalation therapy of asthma using existing dry powder inhaler has a GINA cure rate as low as 5%. The inhaled medication powder particles are prone to congregate and difficult to separate; hard to load enough medication and disperse, resulting in a lactose content as high as 98.8% in the dry powder. However, long-term inhalation of lactose has some side effects; while reducing inhalation of dry powder for alleviating lactose intake can lead to reduction of the medication powder, thereby resulting in poor treatment effect.

Patent document WO 02/089875 A1 discloses a powder deaggregation device (1) which comprises a delivery channel provided at an outlet end with a mouthpiece and an inlet end wherein the inlet end is provided with a deaggregation system comprising a moveable deaggregation element.

Patent document WO 2004/045687 A1 discloses an apparatus for fine powder dispension, such as dispensing micronized pharmaceutical powder during inhalation therapy, has a container for receiving the fine powder and beads with a high speed rotor adapted to receive electrostatic charge and through sharp metal arms to establish an electric field while the arm mechanically operates to develop a cloud of fine particles which is discharged through an outlet mesh which retains the beads in the container.

Patent document WO 2009/022347 A2 discloses a device comprising a convergent nozzle and one filter particularly for containing crushed or sliced fresh garlic.

Patent document US 3,653,380 discloses a dispensing device is described with a mixing chamber in which a dose of powder or other medicament is introduced through an outlet for a human body cavity, such as a mouthpiece or a hollow projection suitable for inserting in a nostril.

Patent document WO 87/ 06475 discloses a particle catcher for reducing the velocity of aerosol medications discharge from an inhalation device and the amount of large particle droplets entrained therein. A screen supportable with respect to the aerosol discharge of the inhalation device is integrally formed with, and along the cross section of, one end of a flexible support tube. The screen includes a structural array of interconnecting elements and openings which reduce the flow rate of the discharged spray and limit the size of particles and droplets in the spray which pass there through. The opposite end of the flexible tube is selectively moldable into the cross-sectional shape of the inhalation device mouthpiece.

Patent document US 6,098,619 discloses an inhaler apparatus having a housing having first and second walls and defining an air inlet and an air outlet. The first and second walls define an internal housing volume and have portions being movable relative to each other so as to expand the internal housing volume from a non-inhalation state, wherein the internal housing volume has a first volume, to an inhalation state, wherein the internal housing volume has a second volume. The second volume is larger than the first volume. An air flow chamber is defined within the housing between the air inlet and the air outlet when the housing is in the inhalation state. A medicament is contained between the first and second walls. The medicament is exposed within the air flow chamber when the housing is in the inhalation state.

### Disclosure of the invention

### Technical problem

The object of the present invention is to improve therapeutic effect by a nozzle that can filter out the medication powder.

### Technology Solutions

The object of the present invention is achieved by: a medication powder nozzle, comprising a suction tube (1) and a filter part (2), wherein, comprising at least one section of hollow suction tube (1), one end of the suction tube (1) is configured as inlet and the other end (16a) configured as outlet, a filter part (2) is provided in the suction tube (1).

In the present invention, the suction tube (1) matches at least one section of subsequent suction tube successively, and the filter part (2) in the outlet way formed by the suction tubes is a filter cup, or a silk screen, mesh, cloth, film, bag in the tubes or the filter part resides anywhere in the outlet way, or other filter parts (2) with the same specification reside in different locations or more than one of filter parts (2) with different specifications are placed respectively.

In the present invention, the filter part (2) comprises at leas two layers of hollow cups, with the slightly bigger one (a) covering the smaller one (b), the convex point (91) of a cup edge matches the other's concave point (92), locking the overlapping positions of the two cups and bifurcating the hollow structures (93) of one cup and the hollow structures (94) of the other cup, however the two cups are communicated via the gap between the overlapping positions of two cups. Given the gap is 10 microns wide, particles below 10 microns can be filtered off from the gap. To prevent the contamination of the cup by hand touching, the wall of the outermost cup is not hollow and several grooves inside the wall go directly to the hollow parts (96) of the bottom of the cup.

In the present invention: a filter bag (2) resides in the filter cup (a), with a support placed in the bag by hooking onto or fixing on to the bag mouth, or the support can lock on or separate from the mouth of the filter cup (a).

In the present invention, mesh number, shape, size, and layer of the filter part (2) are set according to need, for example, allowing particles smaller than 5 microns to pass and blocking those excipients bigger than 6 microns.

In the present invention, said nozzle connects with various types of dry powder inhalers , or at least two said nozzles in parallel connects with various types of dry powder inhalers.

In the present invention, the nozzle connects with various types of dry powder inhalers with, such as dry powder inhaler, medication powder nozzle placement box, receiver device, Turbuhaler, and the inhaler of Chinese Patent No. ZL200510038681.1.

In the present invention: a connecting base (3) residing between the suction tube (a) and the inhaler outlet comprises a center tubular body (31) and a connector (5), the central tubular body (31) inserts into the outlet of the inhalator, and the projections (32, 33) on the central tubular body (31) hooks the inner wall of the outlet, and the connector (5) connects with the suction tube (1), or the connector (5) connects in parallel with at least two suction tubes (1).

In the present invention, a matching inner tube (8) is a hollow tube, whose front end is tapered and rear end is trumpet-shaped chamber in which a sphere body (8a) that can be blocked by a fence (8b) near the chamber port. The front end of the matching inner tube (8) can insert directly into the outlet of the inhaler or insert the central tubular body (31) of the connecting base (3).

In the present invention, insert the connecting base (3) into the outlet of the inhaler, and align the projection (8d) of the matching inner tube (8) to the notch (5a) on the connecting base (3), insert the matching inner tube (8) into the central tubular body (31), lock the position and allow the front arc of the matching inner tube (8) to fit the arc of the outlet of the inhaler, preventing air leakage and facilitating suction of medication powder timely.

### Advantageous Effects

The present invention has the advantage that the powder nozzle can connect with a variety of dry powder inhalers, taking complementary effect and achieving significant effect.

### Description of drawings

Figure 1 shows section view of the suction tube (1) and the filter part (2) according to the present invention.
Figure 2 is a schematic diagram showing appearance of the connecting base (3).
Figure 3 shows section view of the matching inner tube (8), the sphere body (8a) and the fence (8b) according to the present invention.
Figure 4 is a schematic diagram showing the filter part (2): (a) the outer hollow cup (b) inner hollow cup.
Figure 5 shows section view of the nozzle comprised by the suction tube (1) and the suction tube (11), which together with jacket tube (111) connecting to the inhaler.
Figure 6 shows connection between the nozzle of the present invention and the projection (45) on one side at the bottom of the medicine power nozzle placement box.
Figure 7 shows connection of the suction tube (1), suction tube (11), jacket tube (111) and with the connecting base (3).
Figure 8 shows connection of the nozzle and the jacket tube (111), connecting base (3) with the receiver device (103).

### Embodiments of the present invention,

Below in conjunction with the accompanying drawings and embodiments of the present invention will be further described.

### Example 1:

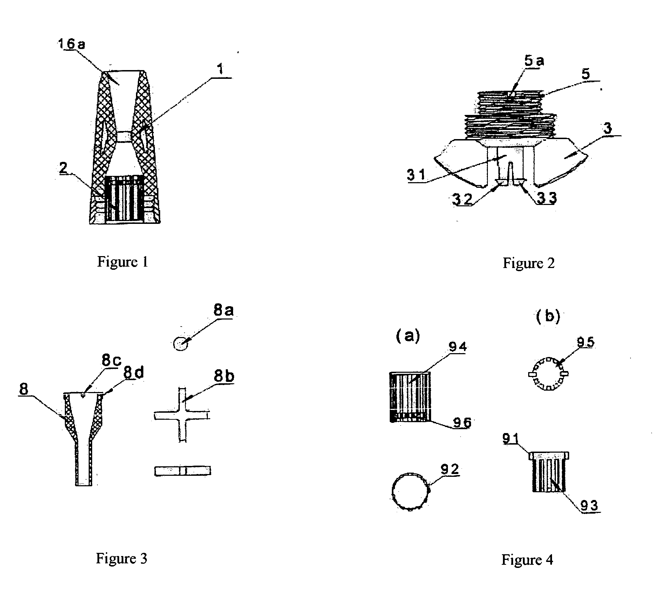

Medication powder nozzle (see Figure 1). Place the inlet of the suction tube (1) upward and install the filter part (2), pour the medication powder into the inlet, and slantwise insert the outlet (16a) of the suction tube (1) into mouth to suck the medication. If the inlet of the suction tube (1) is made curve to upward during the manufacture, the medication will be easy to pour and difficult to overflow. If one end of the outlet (16a) is elongated to 8 cm, the suction rate can be increased. If the outlet (16a) is made of double outlet, nasal use is available. After use, detach and wash the inhalers, blow dry for next use.

### Example 2:

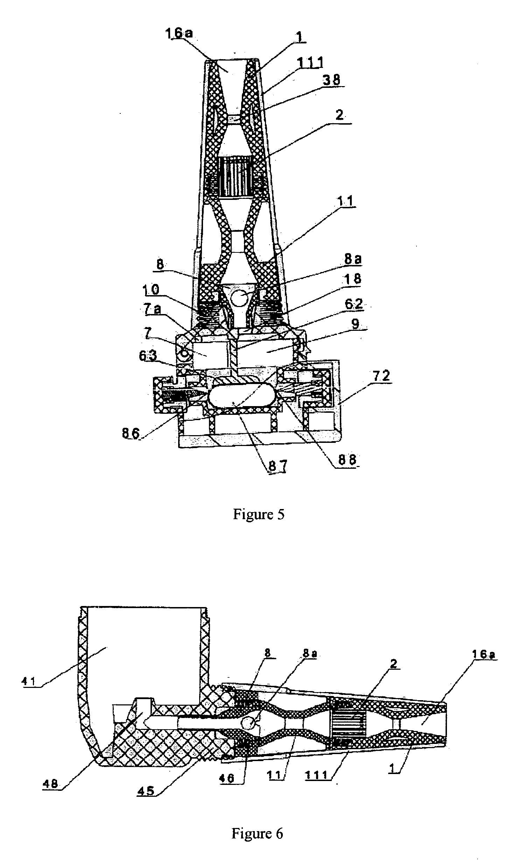

Connect the medicine powder inhaler to the medicine powder nozzle (see Figure 5). Place the filter cup (2) into the chamber of the suction tube (1), and connect the tube (11) with the tube (1) to form a nozzle. Place the sphere body (8a) into the connecting inner tube (8) and fix the sphere body (8a) to the trumpet mouth through the fence (8b); place the connecting inner tube (8) into the outlet (19a) of the medicine powder inhaler, connect the nozzle with the medicine powder connector (10), and cover the nozzle with the jacket tube (111), and connect with the connector (10) forming a new medicine powder inhaler. Open the upper base of the inhaler, place the capsule for inhalation into the capsule chamber, and press the puncture to break up the capsule. When the medication is sucked from the outlet, the sphere body (8a) vibrates to force the excipient to sepatate from the medication powder, and go through the filter cup (2), the excipient is blocked in the filter cup (2), and the filtered medicine powder quickly enters into the human airway. Inadvertent aspiration through the inhaler may cause the sphere body (8a) to block narrow part of the tube, thus preventing the medication powder from being sucked out of inhaler.

### Example 3:

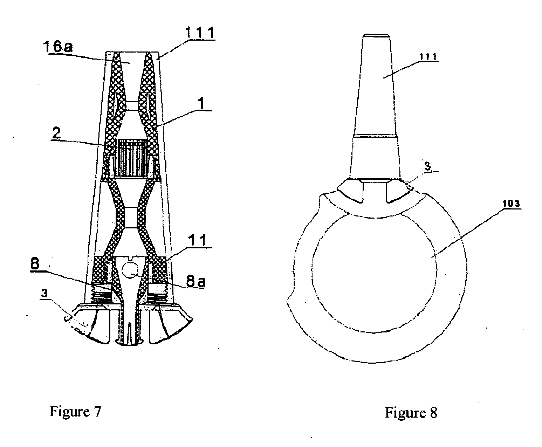

Rotate the Seretide (103) into suction state exposing the outlet, insert the central tubular body (31) on the connecting base (3) into the outlet, align the projection (8d) on the matching inner tube (8) to the notch (5a) on the connecting base (3) and insert into the central tubular body (31), connect the nozzle to the connector (5) on the connecting base (3), finally jacket the jacket tube (111) for use (see Figure 7, Figure 8).

The parts involved by the present invention in embodiment can be made by plastic injection, metal, or new materials. The parts can be produced respectively and then match to each other by connection.

## Claims

1. A medication powder nozzle, comprising a suction tube (1) and a filter part (2), wherein, comprising at least one section of hollow suction tube (1), one end of the suction tube (1) is configured as inlet and the other end (16a) is configured as outlet,
- the filter part (2) is provided in the suction tube (1);
- the filter part (2) comprises at least two layers of hollow-out cups, with the bigger hollow-out cup (a) covering the smaller hollow-out cup (b),
- the two cups are communicated via the gap between overlapping positions of the two cups
**characterized in that** the convex point (91) of the smaller cup's edge matches the bigger cup's concave point (92), locking the overlapping positions of the two cups and bifurcating the hollow structures (93) of the smaller cup and the hollow structures (94) of the bigger cup.

2. The medication powder nozzle according to claim 1, wherein, the suction tube (1) matches at least one section of subsequent suction tube successively, and the filter part (2) in the outlet (16a) way formed by the suction tubes is a filter cup, or a silk screen, mesh, cloth, film, bag in the tubes or the filter part resides anywhere in the outlet (16a) way, or other filter parts (2) with the same specification reside in different locations or more than one of filter parts (2) with different specifications are placed respectively.

3. The medication powder nozzle according to claim 1 or 2, wherein, given the gap between the two cups is 10 microns wide, particles below 10 microns can be filtered off from the gap between the two cups; to prevent the contamination of the bigger cup (a) by hand touching, the wall of the bigger cup (a) is not hollow and several grooves inside the wall go directly to the hollow parts (96) of the bottom of the bigger cup.

4. The medication powder nozzle according to claim 1, 2 or 3, wherein, the filter bag (2) resides inside the filter cup (a), with a support placed in the bag by hooking onto or fixing on to the bag mouth, or the support can lock on or separate from the mouth of the filter cup (a).

5. The medication powder nozzle according to any one of claims 1-4, wherein, mesh number, shape, size, and layer of the filter part (2) are set for allowing particles smaller than 5 microns to pass and blocking excipients bigger than 6 microns.

6. The medication powder nozzle according to any one of claims 1-5, wherein, the nozzle connects with various types of dry powder inhalers, or at least two said nozzles in parallel connects with various types of dry powder inhalers.

7. The medication powder nozzle according to claim 6, wherein, the nozzle connects with various types of dry powder inhalers, such as dry powder inhaler, medication powder nozzle placement box or receiver device.

8. The medication powder nozzle according to claim 6 or 7, wherein, a connecting base (3) residing between the suction tube (1) and the inhaler outlet comprises a center tubular body (31) and a connector (5), the central tubular body (31) inserts into the outlet (16a) of the inhalator, and the projections (32, 33) on the central tubular body (31) hook the inner wall of the outlet, and the connector (5) connects with the suction tube (1), or the connector (5) connects in parallel with at least two suction tubes (1).

9. The medication powder nozzle according to claim 1, 6, 7, or 8, wherein, a matching inner tube (8) is a hollow tube whose front end is tapered and rear end is trumpet-shaped chamber in which a sphere body (8a) that can be blocked by a fence(8b) near the chamber port; the front end of the matching inner tube (8) can insert directly into the outlet of the inhaler or insert into the central tubular body (31) of the connecting base (3).

10. The medication powder nozzle according to claim 8 or 9, wherein, the projection (8d) of the matching inner tube (8) corresponds to a notch (5a) on the connecting base (3), insert the matching inner tube (8) into the central tubular body (31), lock the position and allow the front arc of the matching inner tube (8) to fit the arc of the outlet (16a) of the inhaler.

## Patentansprüche

1. Arzneimittelpulverdüse mit einem Saugrohr (1) und einem Filterteil (2), umfassend mindestens ein Teil von ausgehölten Saugrohr (1), wobei ein Ende des Saugrohrs (1) als Einlass und das andere Ende (16a) als Auslass konfiguriert ist,
- das Filterteil (2) im Saugrohr (1) angeordnet ist;
- das Filterteil (2) mindestens zwei Lagen becherförmiger Elemente mit Aushöhlungen aufweist, wobei das größere becherförmige Element (a) mit Aushöhlungen das kleinere becherförmige Element (b) mit Aushöhlungen abdeckt,
- die beiden becherförmigen Elemente über einen Zwischenraum zwischen Überlappungspositionen der beiden becherförmigen Elemente miteinander kommunizieren;
**dadurch gekennzeichnet, dass**
eine konvexe Stelle (91) des Randes des kleineren becherförmigen Elements einer konkaven Stelle (92) des größeren becherförmigen Elements angepasst ist, wodurch die Überlappungspositionen der beiden becherförmigen Elemente arretiert werden und Ausschnittstrukturen (93) des kleineren becherförmigen Elements und Ausschnittstrukturen (94) des größeren becherförmigen Elements sich gabeln.

2. Arzneimittelpulverdüse nach Anspruch 1, wobei das Saugrohr (1) mindestens einem Teil eines nachfolgenden Saugrohrs angepasst ist, und wobei das Filterteil (2) in dem durch die Saugrohre gebildeten Kanal des Auslasses (16a) ein Filterbecher oder ein Siebelement, ein Maschenmaterial, ein Stoffmaterial, ein Film oder ein Schlauchmaterial in den Rohren ist, oder wobei das Filterteil irgendwo im Kanal des Auslasses (16a) angeordnet ist, oder wobei andere Filterteile (2) mit der gleichen Spezifikation an verschiedenen Stellen angeordnet sind oder wobei mehr als ein Filterteil (2) mit verschiedenen Spezifikationen an jeweiligen Positionen angeordnet sind.

3. Arzneimittelpulverdüse nach Anspruch 1 oder 2, wobei, vorausgesetzt, dass der Zwischenraum zwischen den beiden becherförmigen Elementen 10 µm breit ist, Partikel mit einer Größe von weniger als 10 µm vom Zwischenraum zwischen den beiden becherförmigen Elementen herausgefiltert werden können, um eine Kontamination des größeren becherförmigen Elements (a) durch Berühren mit einer Hand zu verhindern, und wobei die Wand des größeren becherförmigen Elements (a) nicht ausgehöhlt ist und mehrere Nuten im Inneren der Wand sich direkt zu ausgehöhlten Abschnitten (96) des Bodens des größeren becherförmigen Elements erstrecken.

4. Arzneimittelpulverdüse nach Anspruch 1, 2 oder 3, wobei der Filterbeutel (2) im Inneren des becherförmigen Filterelements (a) angeordnet ist, wobei ein Halter im Beutel angeordnet ist, der auf einer Öffnung des Beutels eingehakt oder daran fixiert ist, oder wobei der Halter an der Öffnung des becherförmigen Filterelements (a) befestigt oder davon getrennt werden kann.

5. Arzneimittelpulverdüse nach einem der Ansprüche 1 bis 4, wobei die Maschenweite, -form und -größe und die Lagenmaterialien des Filterteils (2) derart festgelegt werden, dass Partikel mit einer Größe von weniger als 5 µm durchgelassen und Trägerstoffe mit einer Größe von mehr als 6 µm blockiert werden.

6. Arzneimittelpulverdüse nach einem der Ansprüche 1 bis 5, wobei die Düse mit verschiedenartigen Trockenpulverinhalatoren verbindbar ist oder mindestens zwei der Düsen mit verschiedenartigen Trockenpulverinhalatoren parallel verbindbar sind.

7. Arzneimittelpulverdüse nach Anspruch 6, wobei die Düse mit verschiedenartigen Trockenpulverinhalatoren verbindbar ist, wie beispielsweise mit einem Trockenpulverinhalator, einem Arzneimittelpulverdüseneinstellungkasten oder einer Aufnahmeeinrichtung.

8. Arzneimittelpulverdüse nach Anspruch 6 oder 7, wobei eine zwischen dem Saugrohr (1) und dem Inhalatorauslass angeordnete Verbindungsbasis (3) einen mittigen rohrförmigen Körper (31) und einen Verbinder (5) aufweist, wobei der mittige rohrförmige Körper (31) in den Auslass (16a) des Inhalators eingesetzt ist, und wobei Vorsprünge (32, 33) auf dem mittigen rohrförmigen Körper (31) an der Innenwand des Auslasses eingehakt sind, und wobei der Verbinder (5) mit dem Saugrohr (1) verbunden ist oder wobei der Verbinder (5) mit mindestens zwei Saugrohren (1) parallel verbunden ist.

9. Arzneimittelpulverdüse nach Anspruch 1, 6, 7 oder 8, wobei ein passendes Innenrohr (8) ein Hohlrohr ist, dessen vorderes Ende sich verjüngt und dessen hinteres Ende eine trichterförmige Kammer bildet, in der ein durch ein Gitterelement (8b) blockierbarer kugelförmiger Körper (8a) in der Nähe der Kammeröffnung angeordnet ist, wobei das vordere Ende des passenden Innenrohrs (8) direkt in den Auslass des Inhalators oder in den mittigen rohrförmigen Körper (31) der Verbindungsbasis (3) eingesetzt werden kann.

10. Arzneimittelpulverdüse nach Anspruch 8 oder 9, wobei ein Vorsprung (8d) des passenden Innenrohrs (8) einer Nut (5a) auf der Verbindungsbasis (3) zugeordnet ist, das passende Innenrohr (8) in den mittigen rohrförmigen Körper (31) eingesetzt ist, die Position arretiert und ermöglicht wird, dass die vordere Krümmung des passenden Innenrohrs (8) der Krümmung des Auslasses (16a) des Inhalators angepasst ist.

## Revendications

1. Buse de poudre de médicament, comprenant un tube d'aspiration (1) et une partie de filtre (2), dans lequel, comprenant au moins une section de tube d'aspiration creux (1), une extrémité du tube d'aspiration (1) est configurée comme entrée et l'autre extrémité (16a) est configurée comme sortie,
- la partie de filtre (2) est prévue dans le tube d'aspiration (1),
- la partie de filtre (2) comprend au moins deux couches de coupelles creuses, la coupelle creuse la plus grosse (a) recouvrant la coupelle creuse la plus petite (b),
- les deux coupelles sont en communication via l'écart entre des positions de chevauchement des deux coupelles,
**caractérisée en ce que** le point convexe (91) du bord de la coupelle la plus petite correspond au point concave de la coupelle la plus grosse (92), en verrouillant les positions de chevauchement des deux coupelles et en faisant bifurquer les structures creuses (93) de la coupelle la plus petite et les structures creuses (94) de la coupelle la plus grosse.

2. Buse de poudre de médicament selon la revendication 1, dans laquelle le tube d'aspiration (1) correspond à au moins une section du tube d'aspiration suivant successivement, et la partie de filtre (2) dans le moyen de sortie (16a) formé par les tubes d'aspiration est une coupelle de filtre, ou un écran de soie, une maille, un tissu, un film, un sac dans les tubes ou la partie de filtre réside partout dans le moyen de sortie (16a), ou d'autres parties de filtre (2) avec la même spécification résident dans des endroits différents ou plus que l'une des parties de filtre (2) avec des spécifications différentes sont placées respectivement.

3. Buse de poudre de médicament selon la revendication 1 ou 2, dans laquelle, étant donné que l'écart entre les deux coupelles est de 10 microns de large, des particules inférieures à 10 microns peuvent être filtrées à partir de l'écart entre les deux coupelles ; pour éviter la contamination de la coupelle la plus grosse (a) par le toucher de la main, la paroi de la coupelle la plus grosse (a) n'est pas creuse et plusieurs rainures à l'intérieur de la paroi vont directement dans les parties creuses (96) du fond de la coupelle la plus grosse.

4. Buse de poudre de médicament selon la revendication 1, 2 ou 3, dans laquelle, le sac de filtre (2) se trouve à l'intérieur de la coupelle de filtre (a), avec un support placé dans le sac par accrochage sur, ou fixation à, l'embouchure du sac, ou le support peut se verrouiller à, ou se séparer de, l'embouchure de la coupelle de filtre (a).

5. Buse de poudre de médicament selon l'une quelconque des revendications 1 à 4, dans laquelle, le numéro de maille, la forme, la taille et la couche de la partie de filtre (2) sont définis pour permettre à des particules plus petites que 5 microns de passer et bloquer des excipients plus grands de 6 microns.

6. Buse de poudre de médicament selon l'une quelconque des revendications 1 à 5, dans laquelle, la buse se connecte avec différents types d'inhalateurs de poudre sèche, ou au moins deux desdites buses se connectent en parallèle avec les différents types d'inhalateurs de poudre sèche.

7. Buse de poudre de médicament selon la revendication 6, dans laquelle, la buse se connecte avec différents types d'inhalateurs de poudre sèche, tels que un inhalateur de poudre sèche, une boîte de positionnement de buse de poudre de médicament ou un dispositif de réception.

8. Buse de poudre de médicament selon la revendication 6 ou 7, dans lequel, une base de liaison (3) se trouvant entre le tube d'aspiration (1) et la sortie de l'inhalateur comprend un corps central tubulaire (31) et un connecteur (5), le corps central tubulaire (31) s'insère dans la sortie (16a) de l'inhalateur, et les saillies (32, 33) sur le corps central tubulaire (31) sont accrochées sur la paroi interne de la sortie, et le connecteur (5) se raccorde au tube d'aspiration (1), ou le connecteur (5) se connecte en parallèle avec au moins deux tubes d'aspiration (1).

9. Buse de poudre de médicament selon la revendication 1, 6, 7 ou 8, dans laquelle, un tube intérieur correspondant (8) est un tube creux dont l'extrémité antérieure est conique et l'extrémité arrière est une chambre en forme de trompette, dans laquelle un corps de sphère (8a) qui peut être bloqué par un guide (8b) à proximité de l'orifice de chambre ; l'extrémité avant du tube intérieur correspondant (8) peut s'insérer directement dans la sortie de l'inhalateur ou s'insérer dans le corps central tubulaire (31) de la base de liaison (3).

10. Buse de poudre de médicament selon la revendication 8 ou 9, dans laquelle, la saillie (8d) du tube intérieur correspondant (8) correspond à une encoche (5a) sur la base de liaison (3), insérer le tube intérieur correspondant (8) dans le corps central tubulaire (31), verrouiller la position et permettre à l'arc avant du tube intérieur correspondant (8) d'ajuster l'arc de la sortie (16a) de l'inhalateur.
